# FASCICULE DE BREVET EUROPEEN

(11) **EP 1 669 103 B1**
(45) Date de publication et mention de la délivrance du brevet: **08.10.2008**
(21) Numéro de dépôt: 05292116.0
(22) Date de dépôt: 11.10.2005
(51) Int. Cl.: A61Q 1/14, A61Q 5/02, A61Q 19/10, A61K 8/04, A61K 8/31, A61K 8/44

(54) **Composition de nettoyage sous forme de mousse aérosol sans tensioactif anionique et ses utilisations en cosmétique**
Anionisches Tensidfreies Reinigungsmittel in Form eines Aerosol-Schaumes und dessen Verwendungen in Kosmetika
An anionic surfactant free cleansing composition in the form of an aerosol foam and uses thereof in cosmetics

(30) Priorité: 26.11.2004 FR 0452773
(43) Date de publication de la demande: 14.06.2006
(73) Titulaire: L'ORÉAL, 75008 Paris (FR)
(72) Inventeur: Ribery, Delphine, 75013 Paris (FR); Penverne, Isabelle, 95310 St-Ouen L' Aumone (FR)
(74) Mandataire: Miszputen, Laurent

(56) Documents cités:
- EP-A- 0 820 755
- EP-A- 1 340 485
- WO-A-20/04089320
- DE-A1- 19 818 737
- US-A- 5 888 478
- US-A1- 2003 022 799
- US-B1- 6 395 258

## Description

L'invention concerne une composition moussante de nettoyage sans tensioactif anionique comprenant dans un milieu aqueux cosmétiquement acceptable :
a) plus de 2% en poids en matière active d'au moins un tensioactif amphotère ou zwitterionique non-bétainique particulier par rapport au poids total de la composition,
b) au moins un tensioactif non-ionique particulier,
c) au moins un propulseur ; dans une quantité totale de tensioactif(s) amphotère(s) et non-ionique(s) en poids particulière.

L'invention concerne également le dispositif contenant cette composition ainsi que l'utilisation de cette composition en cosmétique ou dermatologie notamment pour le nettoyage des matières kératiniques telles que la peau, les cheveux ou le cuir chevelu.

Il existe actuellement sur le marché de l'hygiène corporelle des gels lavants automoussants conditionnés dans des dispositifs aérosols à 2 compartiments. On connaît également des produits pour la douche conditionnés dans un dispositif aérosol comportant un seul compartiment.

Un des souhaits des consommateurs est de disposer de produits apportant dans un même geste, nettoyage et soin de la peau, tout en profitant du plaisir apporté par une mousse abondante qui se rince facilement. La présentation des produits de nettoyage en aérosol permet, grâce au propulseur de générer rapidement, par action sur un bouton poussoir, une mousse abondante que l'on étale ensuite sur le corps.

La rigidité de la mousse générée à la sortie de l'aérosol est une caractéristique importante. En effet, c'est elle qui va procurer au consommateur, lors de la prise en main et de l'étalement, une sensation de densité et d'effet soin.

Il existe sur le marché des produits pour la douche présentés en aérosol. Ces produits contiennent tous des tensioactifs anioniques qui produisent une mousse de forte densité compacte mais qui présentent l'inconvénient de poser encore en raison de la présence des tensioactifs anioniques des problèmes de tolérance vis de la peau et/ou des cheveux chez certaines personnes ayant la peau atopique ou sensible et / ou sèche.

On a déjà proposé dans le brevet US 6,395,258 de fabriquer des produits de lavage doux peu moussants sous forme aérosol à base de tensioactifs non-ioniques, d'un émollient du type alcool gras, d'un autre type d'émollient, d'un hydratant et d'un propulseur hydrosoluble (oxyde d'azote). Ces produits sont destinés à produire une mousse de très faible densité qui ne nécessite pas de rinçage à l'eau.

On a déjà proposé dans la demande WO00/64404 des shampooings conditionneurs sous forme de mousse aérosol sans tensioactif anionique dont la base lavante contient au moins un tensio-actif non-ionique en particulier du type N-méthyl alkyl alkylglucamide et éventuellement un tensioactif amphotère du type bétaïne.

On connaît également dans la demande DE19818737 un exemple de shampooing conditionneur sous forme de mousse aérosol contenant une base lavante sans tensioactif anionique comprenant 2% de cocoamphoacétate, 3% de cocoamidopropylbétaïne, 5% de laureth-7 et 5% d'alkylpolyglucoside et un système propulseur constitué d'hydrocarbure et de dioxyde de carbone.

On a déjà proposé dans la demande de brevet EP-A-820755 des compositions détergentes post-moussantes capables de produire une mousse instantanée comprenant un premier tensioactif choisi parmi les alkyl éthers sulfates (Sodium, Lauryl Ether Sulfate ou Sodium Trideceth Sulfate) et les amines oxides (Lauryldimethylamine Oxide) ou leurs mélanges, un deuxième tensioactif choisi parmi les amphotères (Cocoamido propyl Betaine ou Cocoamidopropyl Hydroxysultaine), un solvant hydrocarbure volatil, de l'eau et une quantité suffisante de polyethyleneglycol ether ou polypropyleneglycol oleate pour solubiliser ledit hydrocarbure volatil.

On a déjà proposé dans la demande de brevet US2003/0022799 des shampooings moussants capillaires comprenant de 0.005 à 5% en poids d'un polymère cationique, de 10 à 20% en poids d'un tensioactif anionique, de 0,5 à 1,0% en poids d'un sel organique d'acide carboxylique, de 6 à 15% d'un dérivé de sorbitan, de 3 à 6% d'une tensioactif zwitterionique comprenant les dérivés de N, N-dimethylglycine alkyl-susbstitués ou alkyl-substiutués quaternaires, de 0,25 à 5% de tensioactif amphotère, de 0,45 à 1,5% d'un acide carboxylique, 0,1 à 5% de silicone copolyol et éventuellement un propulseur d'aérosol.

On a déjà proposé dans le brevet US5,888,478 des compositions transparentes aérosols comprenant des mélanges de tensioactifs amphotères et de tensioactifs non-ioniques et un tensioactif non-ionique épaississant particulier.

Aucun de ces documents ne se préoccupe de pouvoir produire une mousse rigide abondante facilement rinçable à l'eau qui va procurer au consommateur, lors de la prise en main et de l'étalement, une sensation de densité et d'effet soin.

Il existe donc le besoin de réaliser des compositions de nettoyage doux des matières kératiniques en particulier de la peau sous forme de mousse aérosol dans lesquelles la base lavante ne contient pas de tensioactif anionique et pouvant produire à la sortie du dispositif aérosol une mousse aussi abondante et facilement éliminable à l'eau que celles produites par les compositions détergentes aérosols à base de tensioactifs anioniques actuellement sur le marché sans les inconvénients de tolérance vis-à-vis de la peau et/ou des cheveux énoncés ci-dessus.

La demanderesse a découvert de manière surprenante que l'on pouvait atteindre cet objectif en utilisant une composition comprenant une base lavante constituée de plus de 2% en poids en matière active par rapport au poids total de la composition d'au moins un tensioactif amphotère ou zwitterionique non-bétainique particulier et d'au moins un tensioactif non-ionique particulier, dans une quantité totale de tensioactif(s) amphotère(s) et non-ionique(s) en poids particulière.

Les compositions selon l'invention ainsi obtenues présentent une excellente tolérance vis-à-vis de la peau, des cheveux et des muqueuses.

Elles produisent à la sortie du dispositif aérosol une mousse rigide abondante facilement rinçable à l'eau qui va procurer au consommateur, lors de la prise en main et de l'étalement, une sensation de densité et d'effet soin.

Cette découverte est à la base de la présente invention.

L'invention concerne une composition moussante de nettoyage sans tensioactif anionique comprenant dans un milieu aqueux cosmétiquement acceptable :
a) plus de 2% en poids en matière active d'au moins un tensioactif amphotère ou zwitterionique non-bétainique choisi parmi les alkylamphoacétates, par rapport au poids total de la composition,
b) au moins un tensioactif non-ionique choisi parmi les esters glycérolés du polyéthylèneglycol ,
c) au moins un propulseur ;
la quantité totale de tensioactif(s) amphotère(s) et non-ionique(s) en poids de matière active étant supérieure à 5 % en poids par rapport au poids total de la composition.

L'invention a aussi pour objet un dispositif aérosol comprenant dans un même compartiment la composition moussante.

Un autre objet de l'invention consiste en l'utilisation de ladite composition comme produit de nettoyage et/ou de démaquillage des matières kératiniques.

Un autre objet de l'invention consiste en un procédé de nettoyage des matières kératiniques consistant à produire une mousse à la sortie d'un dispositif aérosol dans lequel est conditionnée la composition de l'invention et à l'appliquer sur lesdites matières kératiniques puis après un éventuel temps de pose, à procéder au rinçage.

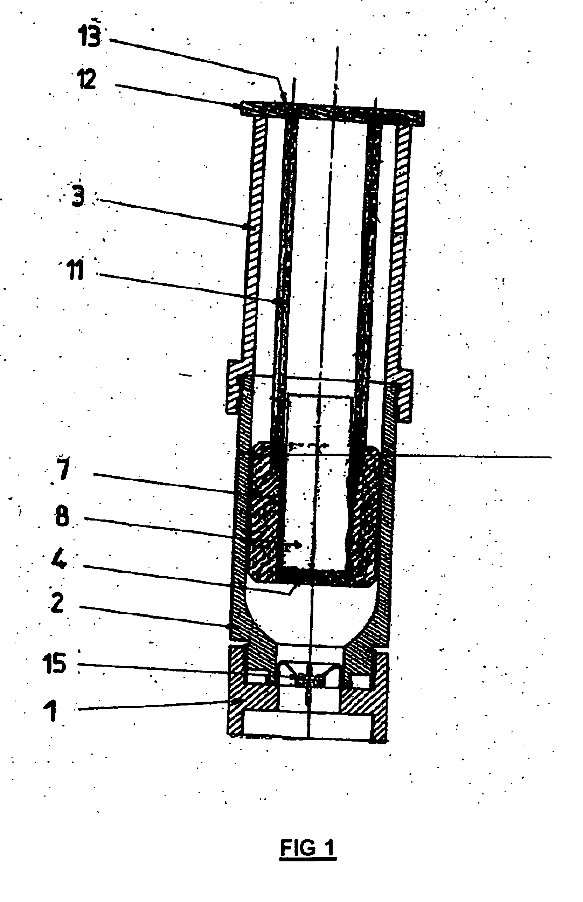

La figure 1 représente un dispositif permettant la mesure de la rigidité d'une mousse.

On entend par « composition sans tensioactif anionique » toute composition contenant moins de 5% en poids de tensioactif anionique, de préférence moins de 1% en poids et plus particulièrement totalement dépourvue de tensioactif anionique.

On entend par « tensioactif amphotère ou zwitterionique bétaïnique », les alkylbétaïnes, les N-alkylamidobétaïnes et leurs dérivés, les sultaïnes ou leurs mélanges

Les matières kératiniques au sens de la présente invention comprennent la peau (corps et visage), le cuir chevelu et les cheveux.

On peut conditionner les compositions de nettoyage de la présente invention dans des dispositifs aérosols en présence de n'importe quel agent propulseur usuellement employé pour la préparation de compositions aérosols. On peut citer en particulier les gaz hydrocarbonés, comme par exemple le propane, le n-butane, l'isobutane et leurs mélanges; les gaz fluorés comme par exemple le chlorodifluorométhane, le dichlorodifluorométhane, le difluoroéthane, le chlorodifluoroéthane, le dichlorotétrafluoroéthane, et leurs mélanges ; les gaz hydrofluorocarbonés; le dimethyl ether et les mélanges de dimethyl ether avec un ou plusieurs gaz hydrocarbonés ; l'azote, l'air et le dioxyde de carbone et leurs mélanges peuvent également être utilisés comme gaz propulseurs dans la présente invention. De façon préférentielle, on utilise dans la présente invention des gaz hydrocarbonés ayant de 2 à 6 atomes de carbone et plus particulièrement un mélange iso-butane, propane, n-butane.

Le ou les gaz propulseurs sont présents dans le dispositif à raison de 0,1 à 15% en poids et plus préférentiellement de 1 à 8% en poids par rapport au poids total de la composition.

La composition de la présente invention conduit à des mousses présentant une rigidité élevée qui procure à l'utilisateur une sensation de densité lors de la prise en main. Les compositions moussantes conformes à l'invention produisent en général à la sortie du dispositif aérosol une mousse ayant une rigidité supérieure à 25s et plus préférentiellement supérieure à 50s, la rigidité étant mesurée selon le dispositif et la méthode suivants.

### Dispositif de mesure de la rigidité

Le dispositif de mesure décrit à la figure 1 comporte un cylindre vertical transparent en plexiglass de diamètre 50 mm, composé de trois parties principales: un socle **1** percé d'un trou de diamètre 24mm sur lequel est posée une valve **15,** un corps inférieur cylindrique **2** de hauteur 104 mm vissé sur le socle, un corps supérieur cylindrique **3** de hauteur 95 mm vissé sur le corps inférieur **2** pour prolonger celui-ci axialement formant ainsi un logement cylindrique vertical ouvert à son extrémité supérieure.

Le dispositif de mesure comporte en outre un mobile coulissant dans le logement cylindrique. Il est composé d'une butée supérieure **12** en PVC de laquelle descendent deux suspentes de piston en inox **11.** La butée **12** est constituée d'un disque de diamètre supérieur à celui du logement cylindrique. Les suspentes sont rattachées à la butée par deux vis **13** et sont enfoncées à leur extrémité inférieure dans un piston **4** en PVC de poids 119,3 g et de diamètre 45 mm. Dans ce piston est disposé un lest en inox **8,** de poids 225 g et de longueur 58,6 mm. Le piston comporte des ailettes de guidage en PVC **7** régulièrement répartis à sa périphérie et s'étendant radialement. Les ailettes sont adaptées pour le guidage du piston suivant l'axe du logement cylindrique.

### Méthode de mesure de la rigidité à partir du dispositif de mesure décrit ci dessus

Un dispositif aérosol à tester est placé une heure et demi dans un bain d'eau à 20 ± 1 °C. Après avoir agité ce dispositif aérosol en faisant cinq allers et retours, celui ci est vidangé à 5 % pour obtenir un taux de remplissage du dispositif aérosol de 95 %. La valve du dispositif aérosol à tester est alors enclenchée sur la valve placée dans le socle du corps inférieur du dispositif de mesure afin de remplir le logement cylindrique de mousse. Cette opération doit être effectuée sans inclure d'air. On arase ensuite la surface supérieure du logement cylindrique. Le corps supérieur est alors vissé au corps inférieur. Le mobile qui comporte le lest est introduit dans le logement cylindrique depuis l'extrémité ouverte. Quand le fond du mobile est en contact avec la mousse, on déclenche le chronomètre. Le chronomètre est arrêté lorsque le mobile a terminé sa descente, c'est-à-dire quand la butée **12** entre en contact avec le sommet du corps supérieur.

La rigidité est mesurée par le temps en seconde que met le mobile pour descendre dans le logement cylindrique.

Les tensioactifs amphotères ou zwitterioniques non-bétainiques conformes à l'invention sont choisis parmi les alkylamphoacétates.

Comme alkylamphoacétates, on peut citer par exemple le N-cocoyl-N-carboxyméthoxyéthyl-N-carboxyméthyl-éthylènediamine N-di-sodique (nom CTFA : disodium cocoamphodiacétate) comme le produit commercialisé sous la dénomination MIRANOL C2M CONCENTRE NP® par la société Rhodia Chimie ; et le N-cocoyl-N-hydroxyéthyl-N-carboxyméthyl-éthylènediamine N-sodique (nom CTFA : sodium cocamphoacétate).

On utilisera plus particulièrement le cocoamphodiacétate de disodium comme le produit commercialisé sous la dénomination commerciale MIRANOL® C2M CONCENTRE NP® (solution à 39% en matière active) par la société RHODIA.

Les compositions selon l'invention contiennent également un ou plusieurs tensioactifs non ioniques choisis parmi les esters glycérolés du polyéthylèneglycol comme par exemple le mélange PEG-7 GLYCERYL COCOATE/ PEG-200 HYDROGENATED GLYCERYL PALMATE (nom CTFA) comme le produit commercial REWODERM LI S80 par la société Degussa Care Specialties.

Selon une forme particulièrement préférée, la composition selon l'invention contient en plus un tensioactif du type bétaïne.

Les bétaïnes sont de préférence choisies parmi les alkylbétaïnes, les N-alkylamidobétaïnes et leurs dérivés, les sultaïnes ou leurs mélanges.

Comme alkylbétaïnes, on peut citer par exemple la cocobétaïne comme le produit commercialisé sous la dénomination DEHYTON AB-30® par la société Cognis ; la laurylbétaïne comme le produit commercialisé sous la dénomination GENAGEN KB® par la société Clariant ; la laurylbétaïne oxyethylénée (10 OE), comme le produit commercialisé sous la dénomination LAURYLETHER (10 OE) BETAINE® par la société Shin Nihon Rica ; la stéarylbétaïne oxyéthylénée (10 OE) comme le produit commercialisé sous la dénomination STEARYLETHER (10 OE) BETAINE® par la société Shin Nihon Rica.

Parmi les N-alkylamidobétaines et leurs dérivés, on peut citer par exemple la cocamidopropyl bétaine commercialisée sous la dénomination LEBON 2000 HG® par la société Sanyo, ou sous la dénomination EMPIGEN BB® par la société Albright & Wilson ; la lauramidopropyl bétaïne commercialisée sous la dénomination REWOTERIC AMB12P® par la société Witco.

Comme sultaines, on peut citer le cocoyl-amidopropylhydroxy-sulfobetaine commercialisé sous la dénomination CROSULTAINE C-50® par la société Croda.

On utilisera plus particulièrement, la cocobétaïne comme le produit commercialisé sous la dénomination DEHYTON AB-30® par la société Cognis

La quantité totale de tensioactif(s) amphotère(s) et non-ionique(s) en poids de matière active est supérieure à 5 % en poids. Elle peut aller par exemple, de 5,1 à 50 % en poids, de préférence de 6 à 50 % en poids, mieux de 6 à 30 % en poids et encore mieux de 8 à 25 % en poids par rapport au poids total de la composition.

Le milieu aqueux cosmétiquement acceptable des compositions de l'invention peut contenir, outre l'eau, un ou plusieurs solvants choisis parmi les alcools inférieurs comportant de 1 à 6 atomes de carbone, tels que l'éthanol ; les polyols tels que la glycérine ; les glycols comme le butylène glycol, l'isoprène glycol, le propylène glycol, les polyéthylène glycols tels que le PEG-8; le sorbitol ; les sucres tels que le glucose, le fructose, le maltose, le lactose, le sucrose ; et leurs mélanges. La quantité de solvant(s) dans la composition de l'invention peut aller de 0,5 à 30 % en poids et de préférence de 5 à 20 % en poids par rapport au poids total de la composition. De préférence, la composition comprend de 50 à 95 % en poids d'eau par rapport au poids total de la composition.

Les compositions de l'invention peuvent comprendre des adjuvants habituellement utilisés dans le domaine cosmétique, et notamment ceux utilisés dans les produits de nettoyage. Comme adjuvants, on peut citer par exemple les parfums, les conservateurs, les séquestrants (EDTA), les pigments, les agents nacrants ou opacifiants, les charges minérales ou organiques, matifiantes, blanchissantes ou exfoliantes, les colorants solubles, les actifs cosmétiques ou dermatologiques, les polymères non ioniques tels que le polyvinylpyrrolidone (PVP), les polymères anioniques, les polymères cationiques, les corps gras comme les huiles ou les cires. Les quantités de ces différents adjuvants sont celles classiquement utilisées dans le domaine considéré, et par exemple de 0,01 à 20 % du poids total de la composition. Ces adjuvants ainsi que leurs concentrations doivent être tels qu'ils ne modifient pas la propriété recherchée pour la composition de l'invention.

Comme exemple d'huile, on peut citer les huiles d'origine végétale (jojoba, avocat, sésame, tournesol, maïs, soja, carthame, pépins de raisin), les huiles minérales (vaseline, isoparaffines éventuellement hydrogénées), les huiles de synthèse (myristate d'isopropyle, octanoate de cétéaryle, polyisobutylène, palmitate d'éthyl-hexyle, alkyl benzoates), les huiles de silicone volatiles ou non volatiles telles que les polydiméthylsiloxanes (PDMS) et les cyclodiméthylsiloxanes ou cyclométhicones, et les huiles fluorées ou fluorosiliconées, ainsi que les mélanges de ces huiles.

Comme polymère cationique, on peut citer les polymères suivants :
Polyquaternium 5 tel que le produit MERQUAT 5 commercialisé par la société CALGON ;
Polyquaternium 6 tel que le produit SALCARE SC 30 commercialisé par la société CIBA, et le produit MERQUAT 100 commercialisé par la société CALGON ;
Polyquaternium 7 tel que les produits MERQUAT S, MERQUAT 2200 et MERQUAT 550 commercialisés par la société CALGON, et le produit SALCARE SC 10 commercialisé par la société CIBA ;
Polyquaternium 10 tel que le produit Polymer JR400 commercialisé par la société AMERCHOL;
Polyquaternium 11 tel que les produits GAFQUAT 755, GAFQUAT 755N et GAFQUAT 734 commercialisés par la société ISP ;
Polyquaternium 15 tel que le produit ROHAGIT KF 720 F commercialisé par la société ROHM;
Polyquaternium 16 tel que les produits LUVIQUAT FC905, LUVIQUAT FC370, LUVIQUAT HM552 et LUVIQUAT FC550 commercialisés par la société BASF ;
Polyquaternium 22 tel que le produit MERQUAT 280 commercialisé par la société CALGON ;
Polyquaternium 28 tel que le produit STYLEZE CC10 commercialisé par la société ISP ;
Polyquaternium 39 tel que le produit MERQUAT PLUS 3330 commercialisé par la société CALGON ;
Polyquaternium 44 tel que le produit LUVIQUAT CARE commercialisé par la société BASF;
Polyquaternium 46 tel que le produit LUVIQUAT HOLD commercialisé par la société BASF ;
Polyquaternium 47 tel que le produit MERQUAT 2001 commercialisé par la société CALGON.

On peut aussi utiliser comme polymère cationique les guars cationiques telles que le produit JAGUAR commercialisé par la société RHODIA.

Comme actifs, on peut utiliser dans la composition de l'invention tout actif habituellement utilisé dans les domaines cosmétique et dermatologique, tels que par exemple les vitamines ou provitamines hydrosolubles ou liposolubles comme les vitamines A (rétinol), C (acide ascorbique), B3 ou PP (niacinamide), B5 (panthénol), E (tocophérol), K1, le bêta-carotène, et les dérivés de ces vitamines et notamment leurs esters ; les stéroïdes comme la DHEA et la 7α-hydroxy DHEA ; les antiseptiques ; les antisébohrréïques et antimicrobiens tels que le peroxyde de benzoyle, l'acide salicylique, le triclosan, le tricarban, l'acide azélaïque ; les hydratants comme la glycérine, l'acide hyaluronique, le pyrrolidone carboxylique acide (PCA) et ses sels, le pidolate de sodium, la sérine, le xylitol, le tréhalose, l'ectoïne, les céramides, l'urée ; les agents kératolytiques et anti-âge tels que les alpha-hydroxyacides comme l'acide glycolique, l'acide citrique, l'acide lactique, les béta-hydroxyacides comme l'acide salicylique et ses dérivés ; les enzymes et co-enzymes et notamment le coenzyme Q10 ; les filtres solaires ; les azurants optiques ; les actifs amincissants comme la caféine, la théophyline, la théobromine, les anti-inflammatoires tels que les acides 18 β glycyrrhétinique et ursolique, et leurs mélanges. On peut utiliser un mélange de deux ou plusieurs de ces actifs. Le ou les actifs peuvent être par exemple présents en une concentration allant de 0,01 à 20 %, de préférence de 0,1 à 10 % et mieux de 0,5 à 5 % du poids total de la composition.

Comme charges, on peut citer les charges minérales telles que le talc ou silicate de magnésium (granulométrie: 5 microns) commercialisé sous la dénomination LUZENAC 15 M00® par la société Luzenac, le kaolin ou silicate d'aluminium comme par exemple celui commercialisé sous la dénomination KAOLIN SUPREME® par la société Imerys, ou les charges organiques telles que l'amidon comme par exemple le produit commercialisé sous la dénomination AMIDON DE MAIS B ® par la société Roquette, les microsphères de Nylon comme celles commercialisées sous la dénomination ORGASOL 2002 UD NAT COS® par la société Atochem, les microsphères à base de copolymère de chlorure de vinylidene/Acrylonitrile/methacrylonitrile enfermant de l'isobutane, expansées comme celles commercialisées sous la dénomination EXPANCEL 551 DE® par la société Expancel. On peut ajouter aussi à la composition de l'invention, des fibres comme par exemple des fibres de nylon (POLYAMIDE 0.9 DTEX 0.3 MM commercialisé par les Etablissements Paul Bonté), des fibres de cellulose ou « Rayonne » (RAYON FLOCK RCISE NOOO3 MO4® commercialisé par la société Claremont Flock Corporation).

Comme agents nacrants ou opacifiants, on peut citer les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les éthers à chaînes grasses tels que par exemple le distéaryléther ou le 1-(hexadécyloxy)-2-octadécanol, les alcools gras en particulier les alcools stéarylique, cétylique, béhénylique et leurs mélanges.

Les compositions moussantes selon l'invention peuvent être utilisées dans les domaines cosmétique et dermatologique, et elles peuvent constituer notamment une composition cosmétique et en particulier des produits de nettoyage ou de démaquillage de la peau (corps, visage, yeux), du cuir chevelu et/ou des cheveux. Elles peuvent être utilisées pour tout type de peau (sèche, normale, mixte ou grasse).

Les compositions de l'invention peuvent notamment être utilisées comme produit pour la douche ; produit pour le bain ; comme produit de nettoyage des mains ; comme shampooing ; comme produit démaquillant des yeux et/ou du visage ou des lèvres.

Un autre objet de l'invention consiste en l'utilisation cosmétique de la composition telle que définie ci-dessus, comme produit de nettoyage et/ou de démaquillage des matières kératiniques humaines et plus particulièrement de la peau.

Les compositions selon l'invention peuvent être aussi utilisées pour le traitement des peaux grasses, notamment en y ajoutant des actifs spécifiques de traitement des peaux grasses, tels que les antiséborrhéiques comme par exemple l'acide salicylique et ses dérivés, l'acide azélaïque, le triclosan, le tricarban, le piroctone olamine, la niacinamide (vitamine PP).

Un autre objet de l'invention est l'utilisation de la composition telle que définie ci-dessus pour la préparation d'une composition destinée au traitement des peaux grasses.

Les compositions selon l'invention présentent un pH final généralement compris entre 3 et 10. De préférence, ce pH est compris entre 4 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide minéral ou organique, de préférence un acide carboxylique tel que par exemple l'acide citrique.

Un autre objet de l'invention consiste en un dispositif aérosol comprenant dans un même compartiment la composition moussante selon la présente invention. Selon un mode de réalisation particulier, le dispositif aérosol de la présente invention est un dispositif à un seul compartiment dans lequel la composition moussante de l'invention est conditionnée.

Un autre objet de l'invention consiste en un procédé cosmétique de nettoyage des résidus de salissure des matières kératiniques humaines, caractérisé par le fait qu'on applique une composition telle que définie précédemment sur lesdites matières kératiniques, en présence d'eau, qu'on applique la mousse formée en sortie de l'aérosol, qu'on masse pour former une mousse et qu'on élimine la mousse formée et les résidus de salissure par rinçage à l'eau.
Les exemples qui suivent servent à illustrer l'invention sans toutefois présenter un caractère limitatif. Les quantités indiquées sont en % en poids sauf mention contraire, et les noms des composés en noms chimiques ou noms CTFA (International Cosmetic Ingredient Dictionary and Handbook) selon les cas.

| | **Ex1** | **Ex2** | **Ex3** |
|---|---|---|---|
| **Ingrédients** | **(hors invention)** | **(hors invention)** | **(invention)** |
| DISODIUM | 0 | 4,794 | 4,794 |
| COCOAMPHODIACETATE | | | |
| PEG-200 HYDROGENATED GLYCERYL PALMATE | 2,3265 | 0 | 2,3265 |
| PEG-7 GLYCERYL COCOATE | 0,6345 | 0 | 0,6345 |
| GLYCERINE | 0,94 | 0,94 | 0,94 |
| CHLORURE DE SODIUM | 0,9165 | 2,7542 | 1,8377 |
| SODIUM GLYCOLATE | 0 | 0,9588 | 0,9588 |
| ISOBUTANE | 3,36 | 3,36 | 3,36 |
| BUTANE | 1,44 | 1,44 | 1,44 |
| PROPANE | 1,2 | 1,2 | 1,2 |
| SEQUESTRANT | qs | qs | qs |
| CONSERVATEURS | qs | qs | qs |
| EAU | qsp 100 | qsp 100 | qsp 100 |

La rigidité de la mousse à la sortie du dispositif aérosol est mesurée selon le dispositif de mesure et la méthode décrite précédemment. Les résultats sont reportés dans le tableau 1 ci dessous.

**Tableau 1**

| **Composition** | **Rigidité (en secondes)** |
|---|---|
| **Exemple 1 avec bétaine sans alkylamphoacétate** | 4 |
| **Exemple 2 sans tensioactif non-ionique** | 9 |
| **Exemple 3 (invention)** | **27** |

On observe avec le dispositif aérosol selon l'invention (exemple 3) contenant l'association tensioactif amphotère non-bétainique / tensioactif non-ionique selon l'invention un effet de synergie au niveau de la rigidité de la mousse par rapport aux dispositifs aérosols de l'art antérieur contenant le tensioactif amphotère non bétainique ou non-ionique seul.

## Revendications

1. Composition moussante de nettoyage sans tensioactif anionique comprenant dans un milieu aqueux cosmétiquement acceptable :
a) plus de 2% en poids en matière active d'au moins un tensioactif amphotère ou zwitterionique non-bétainique choisi parmi les alkylamphoacétates , par rapport au poids total de la composition,
b) au moins un tensioactif non-ionique choisi parmi les esters glycérolés du polyéthylèneglycol,
c) au moins un propulseur;
la quantité totale de tensioactif(s) amphotère(s) et non-ionique(s) en poids de matière active étant supérieure à 5 % en poids par rapport au poids total de la composition.

2. Composition selon la revendication 1, où l'agent propulseur est choisi parmi les gaz hydrocarbonés, les gaz fluorés, les gaz hydrofluorocarbonés, le dimethyl ether, l'azote, l'air et le dioxyde de carbone et leurs mélanges.

3. Composition selon la revendication 2, où l'agent propulseur est choisi parmi les gaz hydrocarbonés ayant de 2 à 6 atomes de carbone.

4. Composition selon la revendication 3, où l'agent propulseur est un mélange iso-butane, propane, n-butane.

5. Composition selon l'une quelconque des revendications 1 à 4, où l'agent propulseur est présent à raison de 0,1 à 15% en poids par rapport au poids total de la composition.

6. Composition selon la revendication 5, où l'agent propulseur est présent à raison de 1 à 8% en poids par rapport au poids total de la composition.

7. Composition selon l'une quelconque des revendications 1 à 6, **caractérisée par le fait qu'**elle produit en général à la sortie du dispositif aérosol une mousse ayant une rigidité supérieure à 25s.

8. Composition selon la revendication 7, **caractérisée par le fait qu'**elle produit à la sortie du dispositif aérosol une mousse ayant une rigidité supérieure à 50 s .

9. Composition selon l'une quelconque des revendications 1 à 8, où le tensioactif amphotère ou zwitterionique est le cocoamphodiacétate de disodium.

10. Composition selon l'une quelconque des revendications 1 à 9, où le tensioactif non-ionique est le mélange PEG-7 GLYCERYL COCOATE/ PEG-200 HYDROGENATED GLYCERYL PALMATE.

11. Composition selon l'une quelconque des revendications 1 à 10, contenant en plus au moins un tensioactif du type bétaïne.

12. Composition selon la revendication 11, où le tensioactif du type bétaïne est choisi parmi les alkylbétaïnes, les N-alkylamidobétaïnes et leurs dérivés, les sultaïnes ou leurs mélanges.

13. Composition selon la revendication 11, où le tensioactif du type bétaïne est la cocobétaïne.

14. Composition selon l'une quelconque des revendications 1 à 13, où la quantité totale de tensioactif(s) amphotère(s) et non-ionique(s) en poids de matière active varie de 5,1 à 50 % en poids, de préférence de 6 à 50 % en poids, mieux de 6 à 30 % en poids et encore mieux de 8 à 25 % en poids par rapport au poids total de la composition.

15. Composition selon l'une quelconque des revendications 1 à 14, comprenant en plus un ou plusieurs adjuvants cosmétiques choisis parmi es parfums, les conservateurs, les séquestrants (EDTA), les pigments, les agents nacrants ou opacifiants, les charges minérales ou organiques, matifiantes, blanchissantes ou exfoliantes, les colorants solubles, les actifs cosmétiques ou dermatologiques, les polymères non ioniques, les polymères anioniques, les corps gras.

16. Utilisation cosmétique d'une composition telle que définie dans l'une quelconque des revendications 1 à 15, comme produit de nettoyage et/ou de démaquillage des matières kératiniques humaines.

17. Utilisation selon la revendication 16, comme produit pour le bain ou la douche.

18. Utilisation selon la revendication 16, comme produit pour le nettoyage des mains.

19. Utilisation selon la revendication 16, comme shampooing.

20. Utilisation selon la revendication 16, comme produit démaquillant des yeux, du visage ou des lèvres.

21. Utilisation de la composition telle que définie dans l'une quelconque des revendications 1 à 15, pour la préparation d'une formulation destinée au traitement des peaux grasses.

22. Procédé cosmétique de nettoyage des résidus de salissure des matières kératiniques humaines, **caractérisé par le fait qu**'on applique une composition telle que définie dans l'une quelconque des revendications 1 à 15 sur lesdites matières kératiniques, en présence d'eau, qu'on applique la mousse formée en sortie de l'aérosol, qu'on masse pour former une mousse et qu'on élimine la mousse formée et les résidus de salissure par rinçage à l'eau.

23. Dispositif aérosol comprenant dans un même compartiment la composition moussante telle que définie selon l'une quelconque des revendications 1 à 15.

24. Dispositif selon la revendication 23 comprenant un seul compartiment dans lequel la composition moussante est conditionnée.

## Claims

1. Foaming cleansing composition without anionic surfactant, comprising, in a cosmetically acceptable aqueous medium:
a) more than 2% by weight in terms of active material of at least one non-betaine amphoteric or zwitterionic surfactant chosen from alkylamphoacetates, relative to the total weight of the composition,
b) at least one non-ionic surfactant chosen from glycerolated esters of polyethylene glycol,
c) at least one propellant;
the total amount of amphoteric and non-ionic surfactant(s) by weight of active material being greater than 5% by weight relative to the total weight of the composition.

2. Composition according to Claim 1, in which the propellant is chosen from hydrocarbon-based gases, fluorinated gases, hydrofluorocarbon-based gases, dimethyl ether, nitrogen, air and carbon dioxide, and mixtures thereof.

3. Composition according to Claim 2, in which the propellant is chosen from hydrocarbon-based gases having from 2 to 6 carbon atoms.

4. Composition according to Claim 3, in which the propellant is a mixture of isobutane, propane and n-butane.

5. Composition according to any one of Claims 1 to 4, in which the propellant is present in a proportion of 0.1 to 15% by weight relative to the total weight of the composition.

6. Composition according to Claim 5, in which the propellant is present in a proportion of 1 to 8% by weight relative to the total weight of the composition.

7. Composition according to any one of Claims 1 to 6, **characterized in that** it produces in general, at the outlet of the aerosol device, a foam having a frigidity of greater than 25 s.

8. Composition according to Claim 7, **characterized in that** it produces, at the outlet of the aerosol device, a foam having a rigidity of greater than 50 s.

9. Composition according to any one of Claims 1 to 8, in which the amphoteric or zwitterionic surfactant is disodium cocoamphodiacetate.

10. Composition according to any one of Claims 1 to 9, in which the non-ionic surfactant is the mixture PEG-7 Glyceryl Cocoate/PEG-200 Hydrogenated Glyceryl Palmate.

11. Composition according to any one of Claims 1 to 10, also containing at least one betaine-type surfactant.

12. Composition according to Claim 11, in which the betaine-type surfactant is chosen from alkyl-betaines, N-alkylamidobetaines and derivatives thereof, sultaines or mixtures thereof.

13. Composition according to Claim 11, in which the betaine-type surfactant is cocobetaine.

14. Composition according to any one of Claims 1 to 13, in which the total amount of amphoteric and non-ionic surfactant(s) by weight of active material ranges from 5.1 to 50% by weight, preferably from 6 to 50% by weight, better still from 6 to 30% by weight, and even better still from 8 to 25% by weight, relative to the total weight of the composition.

15. Composition according to any one of Claims 1 to 14, also comprising one or more cosmetic adjuvants chosen from fragrances, preserving agents, sequestering agents (EDTA), pigments, pearlescent agents or opacifiers, mattifying, whitening or exfoliant, mineral or organic fillers, soluble dyes, cosmetic or dermatological active agents, non-ionic polymers, anionic polymers and fatty substances.

16. Cosmetic use of a composition as defined in any one of Claims 1 to 15, as a cleansing and/or make-up-removing product for human keratin materials.

17. Use according to Claim 16, as a bath or shower product.

18. Use according to Claim 16, as a hand cleansing product.

19. Use according to Claim 16, as a shampoo.

20. Use according to Claim 16, as a make-up-removing product for the eyes, the face or the lips.

21. Use of the composition as defined in any one of Claims 1 to 15, for preparing a formulation for use in the treatment of oily skin.

22. Cosmetic method for cleansing the soiling residues from human keratin materials, **characterized in that** a composition as defined in any one of Claims 1 to 15 is applied to said keratin materials, in the presence of water, **in that** the foam formed at the outlet of the aerosol is applied, **in that** massaging is performed so as to form a foam, and **in that** the foam formed and the soiling residues are removed by rinsing with water.

23. Aerosol device comprising, in the same compartment, the foaming composition as defined in any one of Claims 1 to 15.

24. Device according to Claim 23, comprising a single compartment in which the foaming composition is packaged.

## Patentansprüche

1. Schäumende Reinigungszusammensetzung ohne anionisches Tensid, die in einem kosmetisch akzeptablen Medium enthält:
a) mehr als 2 Gew.-% Wirkstoff aus mindestens einem amphoteren zwitterionischen nicht-betaïnischen Tensid, das unter den Alkylamphoacetaten ausgewählt ist,
b) mindestens ein nichtionisches Tensid, das unter den Glycerylestern von Polyethylenglycol ausgewählt ist,
c) mindestens ein Treibmittel,
wobei die gesamte Gewichtsmenge an amphoterem Tensid oder amphoteren Tensiden und nichtionischem Tensid oder nichtionischen Tensiden des Wirkstoffs mehr als 5 Gew.-% beträgt, bezogen auf das Gesamtgewicht der Zusammensetzung.

2. Zusammensetzung nach Anspruch 1, bei der das Treibmittel unter Kohlenwasserstoffgasen, fluorierten Gasen, partiell fluorierten Kohlenwasserstoffen, Dimethylether, Stickstoff, Luft und Kohlendioxid sowie ihren Gemischen ausgewählt ist.

3. Zusammensetzung nach Anspruch 2, bei der das Treibmittel unter Kohlenwasserstoffgasen mit 2 bis 6 Kohlenstoffatomen ausgewählt ist.

4. Zusammensetzung nach Anspruch 3, bei der das Treibmittel ein Gemisch von Isobutan, Propan und n-Butan ist.

5. Zusammensetzung nach einem der Ansprüche 1 bis 4, bei der das Treibmittel in einem Mengenanteil von 0,1 bis 15 Gew.-% vorliegt, bezogen auf das Gesamtgewicht der Zusammensetzung.

6. Zusammensetzung nach Anspruch 5, bei der das Treibmittel in einem Mengenanteil von 1 bis 8 Gew.-% vorliegt, bezogen auf das Gesamtgewicht der Zusammensetzung.

7. Zusammensetzung nach einem der Ansprüche 1 bis 6, **dadurch gekennzeichnet, dass** sie allgemein am Ausgang der Aerosolvorrichtung einen Schaum mit einer Festigkeit von mehr als 25 s erzeugt.

8. Zusammensetzung nach Anspruch 7, **dadurch gekennzeichnet, dass** sie am Ausgang der Aerosolvorrichtung einen Schaum mit einer Festigkeit von mehr als 50 s erzeugt.

9. Zusammensetzung nach einem der Ansprüche 1 bis 8, bei der das amphotere oder zwitterionische Tensid Dinatriumcocoamphodiacetat ist.

10. Zusammensetzung nach einem der Ansprüche 1 bis 9, bei der das nichtionische Tensid das Gemisch PEG-7 GLYCERYL COCOATE/PEG-200 HYDROGENATED GLYCERYL PALMATE ist.

11. Zusammensetzung nach einem der Ansprüche 1 bis 10, die ferner mindestens ein Tensid vom Betaintyp enthält.

12. Zusammensetzung nach Anspruch 11, bei der das Tensid vom Betaïntyp unter Alkylbetaïnen, N-Alkylamidobetaïnen und ihren Derivaten, Sultainen oder ihren Gemischen ausgewählt ist.

13. Zusammensetzung nach Anspruch 11, bei der das Tensid vom Betaïntyp Cocobetaïn ist.

14. Zusammensetzung nach einem der Ansprüche 1 bis 13, bei der die gesamte Gewichtsmenge an amphoterem Tensid oder amphoteren Tensiden und nichtionischem Tensid oder nichtionischen Tensiden des Wirkstoffs im Bereich von 5,1 bis 50 Gew.-%, bevorzugt im Bereich von 6 bis 50 Gew.-%, besser im Bereich von 6 bis 30 Gew.-% und noch besser im Bereich von 8 bis 25 Gew.-% liegt, bezogen auf das Gesamtgewicht der Zusammensetzung.

15. Zusammensetzung nach einem der Ansprüche 1 bis 14, die ferner einen oder mehrere kosmetische Hilfsstoffe enthält, die ausgewählt sind unter Parfüms, Konservierungsmitteln, Sequestrierungsmitteln (EDTA), Pigmenten, Perlglanzmitteln oder Trübungsmitteln, anorganischen oder organischen Füllstoffen, Mattierungsmitteln, Aufhellungsmitteln oder Exfoliationsmitteln, löslichen Farbstoffen, kosmetischen oder dermatologischen Wirkstoffen, nichtionischen Polymeren, anionischen Polymeren und Fettstoffen.

16. Kosmetische Verwendung einer Zusammensetzung wie in einem der Ansprüche 1 bis 15 definiert als Produkt zur Reinigung und/oder zum Abschminken von menschlichen Keratinmaterialien.

17. Verwendung nach Anspruch 16 als Produkt für Bad oder Dusche.

18. Verwendung nach Anspruch 16 als Produkt zur Reinigung der Hände.

19. Verwendung nach Anspruch 16 als Shampoo.

20. Verwendung nach Anspruch 16 als Produkt zum Abschminken der Augen, des Gesichts oder der Lippen.

21. Verwendung der Zusammensetzung wie in einem der Ansprüche 1 bis 15 definiert zur Herstellung einer Formulierung, die zur Behandlung von fetter Haut bestimmt ist.

22. Kosmetisches Verfahren zur Reinigung von menschlichen Keratinmaterialien von Schmutzrückständen, **gekennzeichnet durch** Aufbringen einer Zusammensetzung wie in einem der Ansprüche 1 bis 15 definiert auf die Keratinmaterialien in Gegenwart von Wasser, Aufbringen des am Ausgang der Aerosolvorrichtung gebildeten Schaums, Massieren zur Erzeugung eines Schaums und Entfernen des gebildeten Schaums und der Schmutzrückstände **durch** Spülen mit Wasser.

23. Aerosolvorrichtung, die im gleichen Compartment die schäumende Zusammensetzung enthält, wie sie in einem der Ansprüche 1 bis 15 definiert ist.

24. Vorrichtung nach Anspruch 23, die ein einziges Compartment aufweist, in dem die schäumende Zusammensetzung konfektioniert ist.
